# EUROPÄISCHE PATENTSCHRIFT

(11) **EP 3 944 832 B1**
(45) Veröffentlichungstag und Bekanntmachung des Hinweises auf die Patenterteilung: **12.03.2025**
(21) Anmeldenummer: 20188543.1
(22) Anmeldetag: 30.07.2020
(51) Int. Cl.: A61B 34/00, A61B 90/00, A61B 5/00

(54) **SYSTEM UND VERFAHREN ZUR ERZEUGUNG VON SCHNITTLINIEN**
SYSTEM AND METHOD FOR CREATING CUTTING LINES
SYSTÈME ET PROCÉDÉ DE GÉNÉRATION DE LIGNES DE DÉCOUPE

(43) Veröffentlichungstag der Anmeldung: 02.02.2022
(73) Patentinhaber: Ellicut UG (haftungsbeschränkt), 81667 München (DE)
(72) Erfinder: Derendorf, Stefanie, 81667 München (DE)
(74) Vertreter: Klöckner, Christoph

(56) Entgegenhaltungen:
- EP-A2- 2 389 573
- US-A1- 2007 173 946
- US-A1- 2011 184 291
- US-A1- 2012 019 511
- US-A1- 2015 366 628
- US-A1- 2018 132 726

## Beschreibung

### Technisches Gebiet

Die Erfindung betrifft ein System, um Schnittlinien für die Durchführung eines operativen Eingriffs zur Verfügung zu stellen. Daneben betrifft die Erfindung ein Verfahren sowie ein Computerprogramm, um Schnittlinien zur Verfügung zu stellen.

Operative Therapieverfahren an der Haut gehören zum üblichen Behandlungsspektrum eines Arztes, insbesondere eines Dermatologen. Ein Ziel einer solchen dermatochirurgischen Behandlung ist eine indikationsgerechte und schonende Therapie unter ästhetischen Aspekten.

Die Wahl der optimalen Exzisions- und Rekonstruktionstechnik sowie die Präparation und Markierung von chirurgischen Schnittkanten sind essentiell für eine sichere chirurgische Exzision des betroffenen Gewebes, sowie für eine Minimierung der Traumatisierung, eine optimale Wundheilung und nicht zuletzt zur Vermeidung von Narben unter ästhetischen Gesichtspunkten.

Bei pigmentierten Hautveränderungen wie etwa dysplastischen Naevi (atypischen Muttermalen) und Melanomen sowie bei Nicht-Melanom-Hautkrebs, einschließlich Basalzellkarzinomen und Plattenepithelkarzinomen, wird typischerweise eine sogenannte elliptische Exzision der Haut durchgeführt.

Daneben kann bei klinischem Verdacht auf Krebs eine elliptische Exzision auch als einstufige diagnostische und therapeutische Intervention dienen. Die durch elliptische Exzision gewonnenen Proben ermöglichen eine histopathologische Untersuchung der Haut, einschließlich der Epidermis, Dermis und des subkutanen Fettgewebes. Das Verfahren ermöglicht die Bestimmung der Tiefe der Hautveränderung sowie den Nachweis klarer Ränder innerhalb des umgebenden Gewebes. Vorliegend werden unter Hautveränderungen auch Muttermale, Warzen, maligne und benigne Hauttumoren und weitere Arten von Hautveränderungen gefasst.

Üblicherweise wird bei einer klassischen Vorbereitung mit einem Markierungsstift von einem Arzt, insbesondere Dermatologen, händisch/manuell die Exzision markiert bzw. gestaltet. Ein als Standarddesign zu bezeichnende Gestaltung/Design berücksichtigt dabei insbesondere ein Längen-Breiten-Verhältnis von 3:1 und einen apikalen Winkel von weniger als 30 Grad. Dieses (Standard-) Design wird wegen der scharfen (nicht stumpfen) Spitzen an jedem Ende als fusiforme Ellipse bezeichnet, wobei diese fusiforme Ellipse in Richtung der entspannten Hautspannungslinien ausgerichtet sein sollte.

Der markierte Bereich der geplanten Exzision hat dabei einen direkten Bezug zu einer sicheren Trennung der markierten fusiformen Ellipse und des darunterliegenden Gewebes, was relevant für die Histologie sowie für traumatische Hautverletzung und Vernarbung ist. US 2011/184291 A1 bezieht sich auf ein Ultraschalldiagnosegerät, ein medizinisches Bilddiagnosegerät, ein Ultraschallbildverarbeitungsgerät, ein medizinisches Bildverarbeitungsgerät, ein Ultraschalldiagnosesystem und ein medizinisches Bilddiagnosesystem, die verwendet werden, wenn ein Operationszielbereich oder ein Behandlungszielbereich vor einer chirurgischen Operation oder Behandlung markiert werden soll.

Allerdings hängt aktuell die Wahl der optimalen Exzisionstechnik, die Dimensionierung und Morphologie der Schnittform und die Markierung der gewünschten Schnitte auf der Haut des betroffenen Patienten vom Wissen, der Routine sowie dem handwerklichen Geschick des durchführenden Arztes, Operateurs bzw. Dermatologen als medizinisches Fachpersonal ab. Die gewünschten Schnittkanten werden in der Regel freihändig mit einem Stift auf die Haut aufgetragen. Es gibt zudem keine Möglichkeit die Rekonstruktion, die Nahttechnik und Wundheilung vor dem Eingriff zu simulieren. Einmal auf die Haut aufgetragen, ist es zudem schwierig die händische Markierung zu korrigieren.

Mit anderen Worten sind gegenwärtig sowohl weder Systeme oder Vorrichtungen noch Verfahren für ein medizinisches Fachpersonal verfügbar, welche Schnittlinien gemäß einem standardisierten Prozess erstellen und einem medizinischen Fachpersonal vermitteln und zur Verfügung stellen könnte.

### Zusammenfassung der Erfindung

Es ist die Aufgabe der Erfindung die Nachteile aus dem Stand der Technik zu vermeiden oder wenigstens zu mindern und insbesondere eine Vorrichtung und ein Verfahren zur Verfügung zu stellen, die / das Schnittlinien, insbesondere eine Schnittform, für einen operativen Eingriff, insbesondere eine Exzision, vorzugsweise eine elliptische Exzision, medizinischem Fachpersonal wie etwa einem Arzt, Operateur oder Dermatologen, zur Verfügung stellen kann, so dass eine Behandlung unterstützt und insbesondere noch einfacher und besser durchgeführt werden kann.

Insbesondere ist eine weitere Aufgabe der Erfindung dem medizinischen Fachpersonal ein einfaches sowie einfach zu bedienendes, kostengünstiges und zuverlässiges System sowie Verfahren an die Hand zu geben. Auch soll das System vorzugsweise handlich und lernfähig sein als auch portabel einsetzbar sein.

Die Aufgabe wird hinsichtlich einer gattungsgemäßen Vorrichtung bzw. eines gattungsgemäßen Systems erfindungsgemäß gelöst durch ein System zur Erzeugung von Schnittlinien mit den Merkmalen des Anspruchs 1, hinsichtlich eines gattungsgemäßen Verfahrens erfindungsgemäß durch die Merkmale des nebengeordneten Verfahrensanspruchs 8 und hinsichtlich eines Computerprogramms erfindungsgemäß durch die Merkmale des Anspruchs 9 gelöst.

Konkret wird die Aufgabe der Erfindung gelöst durch ein System zur Erzeugung von Schnittlinien für die Durchführung eines operativen Eingriffs und Darstellung dieser vorgegebenen Schnittlinien auf der Hautoberfläche eines Patienten, insbesondere von vorgegebenen Schnittlinien für eine chirurgische Exzision, umfassend: eine (elektromagnetische) Erfassungsvorrichtung, welche zum digitalen Erfassen, vorzugsweise optischen Erfassen, eines Bereichs der Hautoberfläche eines Patienten mit einer Hautveränderung vorgesehen und angepasst ist, außerdem eine Bestimmungsvorrichtung, die dafür vorgesehen und angepasst ist, anhand des von der Erfassungsvorrichtung erfassten Bereichs der Haut mit der Hautveränderung Schnittlinien, vorzugsweise ein Schnittmuster/eine Schnittform mit chirurgischen Schnittlinien, zur Entfernung der Hautveränderung zu bestimmen, und zuletzt eine Ausgabevorrichtung, die dafür vorgesehen und angepasst ist, die von der Bestimmungsvorrichtung bestimmten Schnittlinien auszugeben und eine Darstellung der Schnittlinien auf der Hautoberfläche zu ermöglichen, dadurch gekennzeichnet, dass das System ferner einen vordefinierten Satz an vorgefertigten, eindeutig nummerierten Schablonen aufweist, wobei: die Bestimmungsvorrichtung ferner dafür angepasst ist, aus dem Satz der nummerierten Schablonen die zu verwendende Schablone zu bestimmen, welche zu den bestimmten vorgegebenen Schnittlinien die geringsten Abweichungen aufweist, und die Ausgabevorrichtung dafür angepasst ist, mittels einer Anzeige die eindeutige Nummer der zu verwendenden Schablone anzuzeigen. Durch dieses dreigliedrige bzw. dreistufige System mit Erfassungsvorrichtung, Bestimmungsvorrichtung und Ausgabevorrichtung ist das System dafür vorgesehen und angepasst eine individuelle Hautveränderung eines Patienten zunächst zu erfassen, intern, insbesondere über eine Recheneinheit oder ECU, diesen erfassten Bereich der Hautoberfläche mit entsprechender Hautveränderung zu analysieren und (digitale) Schnittlinien bzw. ein (digitales) Schnittmuster bzw. eine Schnittform mit chirurgischen Schnittlinien zur Entfernung dieser Hautveränderung zu bestimmen und schließlich einem medizinischen Fachpersonal, wie etwa einem Arzt, Dermatologen oder Operateur, diese individualisierten, auf den Bereich der Hauptoberfläche des Patienten mit der Hautveränderung angepassten Schnittlinien eine Vermittlung bzw. Ausgabe dieser bestimmten bzw. berechneten individuell angepassten Schnittlinien zu ermöglichen. Die (elektromagnetische) Erfassungsvorrichtung ist dabei angepasst, elektromagnetische Wellen, wie etwa Licht, IR und/oder auch Röntgenstrahlung, digital zu erfassen und entsprechend digital weiterzuleiten. Die Ausgabe der bestimmten Schnittlinien auf der Hautoberfläche kann sowohl digital oder virtuell als auch physisch erfolgen.

Dementsprechend wird mit der vorliegenden Erfindung ein System bereitgestellt, welches mithilfe einer digitalen Erfassung, insbesondere einer optischen Erfassung, der betroffenen Hautstelle des jeweiligen Patienten, eine optimale, personalisierte Schnittform für eine Exzision erstellt und durch ein medizinisches Fachpersonal ausgewählt, geplant und simuliert werden kann.

Des Weiteren ist es mithilfe des Systems möglich, Rekonstruktionen, Nahtechniken, sowie mögliche Wundheilungsverläufe zu simulieren und mit dem Patienten im Vorfeld der möglichen operativen Behandlung zu besprechen. Dabei wird ein besonderes Augenmerk auf die späteren Schnittkanten gelegt. Die zu entfernenden Hautteile und Gewebe können insbesondere durch das erfindungsgemäße System bemaßt (wie etwa Fläche, Länge, Winkel, Sicherheitsabstand) und abschließend durch das medizinische Fachpersonal bewertet werden. Insbesondere kann durch das System bereits vorab eine Bewertung und Auswahl von besonders geeigneten (chirurgischen) Schnittlinien bzw. Schnittmustern durchgeführt werden.

Durch das erfindungsgemäße System wird dem medizinischen Fachpersonal eine Vorrichtung zur Verfügung gestellt, welches aufgrund der entsprechend angepassten Bestimmungseinheit eine Art vorbestimmten Prozess zur Ermittlung bzw. Bestimmung von Schnittlinien bereitstellt.

Vorteilhafte Ausführungsformen sind in den Unteransprüchen beansprucht und werden nachfolgend erläutert.

Die Ausgabevorrichtung kann einen Projektor aufweisen und die Ausgabevorrichtung kann weiter dafür angepasst sein, mittels des Projektors die bestimmten, vorgegebenen Schnittlinien mittelbar über eine transparente Scheibe oder unmittelbar auf den erfassten Bereich der Haut des Patienten optisch zu projizieren. Insbesondere ist die Ausgabevorrichtung dafür angepasst, über die Erfassungsvorrichtung selbst oder über eine weitere Kamera, insbesondere eine 3D-Kamera, den Bereich der Haut mit der Hautveränderung räumlich zu erfassen und direkt auf diesen Bereich der Hautveränderung relativ dazu die bestimmten Schnittlinien zu projizieren. Bewegt sich der Patient, so bewegen sich die Schnittlinien entsprechend mit, so dass die Schnittlinien relativ zu der Hautveränderung stets beibehalten werden. Ein Projektor ist ein einfaches und kostengünstiges Mittel, um grafisch die Schnittlinien anzuzeigen. Auch ist von Vorteil, dass sowohl das medizinische Fahrpersonal als auch der Patient gleichzeitig die Schnittlinien sehen können. Werden die Schnittlinien nicht unmittelbar auf die Haut sondern mittelbar auf eine transparente oder milchige oder halbtransparente Scheibe optisch projiziert, so kann insbesondere sichergestellt werden, dass stets die vorbestimmte Ebene der Scheibe als Projektions(ober)fläche genutzt wird, was eine Komplexität des Systems herabsetzt. Auch kann beispielsweise der Projektor, wenn beispielsweise ein Patient nicht vor Ort ist, die Schnittlinien auf die Scheibe projizieren und das medizinische Fachpersonal diese Schnittlinien dann gemeinsam bewerten. Insbesondere ist der Projektor dafür angepasst, eine Überlagerung von dem erfassten Bild des Hautbereichs und den Schnittlinien anzuzeigen, um eine digitale oder virtuelle Darstellung der Schnittlinien auf der digital erfassten Hautoberfläche zu ermöglichen.

Die projizierten Schnittlinien können, zumindest vorübergehend, nach Beendigung der Projektion noch auf dem erfassten Bereich der Hautoberfläche des Patienten erkennbar sein. Die Ausgabevorrichtung ist also angepasst, elektromagnetische Strahlen definierter Wellenlänge, insbesondere unterhalb und/oder oberhalb der Wellenlänge des sichtbaren Lichts, definiert auf einen Bereich der Hautoberfläche zu projizieren. Insbesondere kann die Ausgabevorrichtung dafür angepasst sein, Strahlen, wie etwa UV (Ultraviolett)-Strahlen, definiert ausgerichtet auszusenden (in Art eines Laserstrahls oder einer Projektion mit definiertem Umriss). So kann auf der Haut des Patienten mit der Hautveränderung beispielsweise eine strahlungsempfindliche, insbesondere UV-empfindliche, Substanz aufgebracht werden, auf die die Ausgabevorrichtung in Art einer strahlentechnischen Markierung die Schnittlinien einbringt. Diese strahlungsempfindliche Substanz kann sich beispielsweise durch den Strahleneintrag, insbesondere UV-Eintrag, verfärben, so dass, insbesondere bei einer transparenten Substanz, die Schnittlinien farblich sichtbar werden. Alternativ oder zusätzlich kann auch eine auf die Haut aufgebrachte lose Substanz, beispielsweise ein Pulver, durch den Strahleneintrag verfestigt werden, so dass der verfestigte Bereich die Schnittlinien angibt (in Art eines Sinterns). Die Ausgabevorrichtung kann einen steuerbaren Laser aufweisen, der die Schnittlinien projiziert. Auch kann eine Art, insbesondere halbtransparente oder transparente, strahlungssensitive Folie bereitgestellt werden, die auf den Hautbereich aufbringbar ist, wobei die Ausgabevorrichtung dafür angepasst ist, dass die Wellenlängen der projizierten Strahlen der sensitiven Wellenlänge der strahlungssensitiven Folie entspricht. Mit anderen Worten können dadurch ähnlich eines Foto-Negativs eines fotografischen Films in die strahlungssensitive Folie durch die Ausgabevorrichtung, insbesondere durch den Projektor, die bestimmten Schnittlinien temporär oder permanent eingebracht werden. Das System weist in dieser speziellen Form also neben der Erfassungsvorrichtung, der Bestimmungsvorrichtung und der Ausgabevorrichtung, ferner die an die Ausgabevorrichtung angepasste strahlungssensitive (auf die Haut aufbringbare, insbesondere auflegbare und fixierbare) Folie auf.

Die Ausgabevorrichtung kann ein Display aufweisen und die Ausgabevorrichtung dafür angepasst sein, auf dem Display eine Überlagerung des erfassten optischen digitalen Bildes der Haut des Patienten mit der Hautveränderung sowie der hierzu bestimmten Schnittlinien anzuzeigen, wobei es sich bei dem Display um ein Augmented Reality (AR)-Display handeln kann. Insbesondere kann auf dem Display auch eine Benutzeroberfläche angezeigt werden bzw. ist das Display dafür angepasst eine solche anzuzeigen. Das Display kann ein Touchdisplay sein. Ein solches Display kann beispielsweise ein Computerdisplay/Bildschirm sein oder ein Display eines Hand-Held-Device wie etwa eines Smartphones. Insbesondere kann auch ein Smartphone mittels Kamera, insbesondere 3D-Kamera, und Augmented Reality auf seinem Display eine Überlagerung von der Haut des Patienten mit der Hautveränderung und den bestimmten Schnittlinien anzeigen.

Die Ausgabevorrichtung kann einen Drucker, insbesondere einen 3D-Drucker aufweisen, wobei die Ausgabevorrichtung dafür angepasst ist, mittels des Druckers eine Folie mit den vorgegebenen Schnittlinien oder eine individualisierte Schablone der vorgegebenen Schnittlinien auszudrucken, die auf den erfassten Bereich der Haut mit der Hautveränderung auflegbar und insbesondere auf dem Bereich der Haut fixierbar ist. Dadurch, dass die Ausgabevorrichtung den Drucker aufweist, kann insbesondere auf eine transparente oder halbtransparente Aufklebefolie mit vorzugsweise einer Positions- und/oder Lagemarkierung, eine grafische Schnittliniendarstellung aufgedruckt werden.

Diese Folien können auf insbesondere eine entsprechend komplementäre Positions- und/oder Lagemarkierung auf der Haut des Patienten aufgeklebt werden, sodass die Schnittlinien relativ zu der Hautveränderung direkt auf dieser und angepasst zu dieser aufliegen. Im Falle eines 3D-Druckers kann insbesondere eine individualisierte Schablone ausgedruckt werden, beispielsweise mittels Rapid Prototyping (als additive Fertigung aus dem 3D-Drucker), welche anschließend auf die Stelle der Hautveränderung aufgelegt werden kann.

Die Ausgabevorrichtung kann einen steuerbaren Laser aufweisen und die Ausgabevorrichtung kann dafür angepasst sein, den Laser so zu steuern, dass er aus einem Rohobjekt, wie etwa einer Kunststoffplatte, eine Schablone mit den bestimmten Schnittlinien ausschneidet. Diese Ausführungsform kann auch besonders kompakt und insbesondere portabel gestaltet werden. So kann das System insbesondere eine plane oder eine gekrümmte und an einen Körperabschnitt angepasste, Kunststoffplatte als Rohobjekt vor Ort und schnell herstellen, welche das medizinische Fahrpersonal anschließend für eine Beratung oder operativen Eingriff verwenden kann. Alternativ kann mittels des Lasers auch ein farbiges, insbesondere steriles, Blatt oder eine intransparente, insbesondere sterile, Folie derart ausgeschnitten werden, dass eine definierte Öffnung verbleibt, welche die definierten Schnittlinien darstellen. Mit anderen Worten wird ein mittiger Bereich des Blatts oder der Folie ausgeschnitten, welche dann in Art eines sterilen Operationstuchs auf den Bereich der Haut mit der Hautveränderung aufgebracht werden kann.

Gemäß der vorliegenden Erfindung weist das System ferner einen vordefinierten Satz/Set an vorgefertigten, vorzugsweise desinfizierbaren, eindeutig nummerierten Schablonen auf, wobei: die Bestimmungsvorrichtung ferner dafür angepasst ist, aus dem Satz der nummerierten Schablonen die zu verwendende Schablone zu bestimmen, welche zu den bestimmten vorgegebenen Schnittlinien die geringsten Abweichungen aufweist, und die Ausgabevorrichtung ist dafür angepasst, mittels einer Anzeige, insbesondere mittels eines Displays die eindeutige Nummer der zu verwendenden Schablone anzuzeigen. Hierdurch wird ein besonderes robustes und einfaches System zur Verfügung gestellt. Der vordefinierte Satz an Schablonen kann beispielsweise in Arztpraxen vorgehalten werden und das System bestimmt aus diesem vordefinierten Satz eine geringe Anzahl an geeignetsten oder eine einzige, also die geeignetste Schablone für einen operativen Eingriff.

Insbesondere können die vorgefertigten, eindeutig nummerierten Schablonen auf den erfassten Bereich der Haut mit der Hautveränderung auflegbar und insbesondere auf dem Bereich der Haut fixierbar sein. Insbesondere können die Schablonen eine haftende Oberfläche, beispielsweise eine klebende Oberfläche, aufweisen, so dass diese, aufgelegt auf die Haut des Patienten, an ihrer Stelle stoffschlüssig fixiert haften bleiben. Alternativ oder zusätzlich können die Schablonen eine formschlüssige Besfestigungsvorrichtung, etwa in Form eines elastischen Gummibands, aufweisen, um die Schablonen gegenüber der Haut des Patienten zu fixieren.

Gemäß einer Ausführungsform kann das System ferner eine Eingabevorrichtung, insbesondere in Form einer Tastatur, eines grafischen Zeigergeräts wie etwa einer Maus, eines Touchscreens und/oder einer Gestensteuerung aufweisen, die dafür angepasst ist, weitere medizinische Parameter des Patienten zu erfassen und diese Parameter an die Bestimmungsvorrichtung weiterzuleiten, wobei die Bestimmungsvorrichtung diese Parameter bei der Bestimmung der Schnittlinien berücksichtigt. Die Eingabevorrichtung ermöglicht einen aktiven Eingriff durch das medizinische Fachpersonal. Beispielsweise kann der Arzt ein Alter des Patienten, ein Geschlecht des Patienten, einen Hauttyp des Patienten oder eine Vorerkrankung eingeben, welche von dem System entsprechend berücksichtigt wird, beispielsweise indem bei einem höheren Alter eine Dimensionierung eines Sicherheitsabstandes größer gewählt wird.

Vorzugsweise kann ein, insbesondere portabler, Client, wie etwa ein Hand-Held-Device, die Erfassungsvorrichtung und/oder die Ausgabevorrichtung aufweisen und ein zentraler Server die Bestimmungsvorrichtung aufweisen, wobei die Erfassungsvorrichtung, die Ausgabevorrichtung und die Bestimmungsvorrichtung über ein Netzwerk datentechnisch miteinander in Verbindung stehen, um digitale Daten auszutauschen. Insbesondere stehen diese über ein kabelgebundenes Netzwerk, WLAN und/oder Bluetooth Netzwerk miteinander in Verbindung. Weiter vorzugsweise sind Server und Client über das Internet als zumindest Teilabschnitt des Netzwerks miteinander verbunden.

Gemäß einer Ausführungsform kann die Bestimmungsvorrichtung zur Bestimmung der Schnittlinien dafür angepasst sein, vorgegebene Regeln anzuwenden, um anhand des von der Erfassungsvorrichtung erfassten Bereichs der Haut mit der Hautveränderung Schnittlinien zur Entfernung der Hautveränderung zu bestimmen. Diese Regeln können insbesondere als Standardregeln hinterlegt sein und/oder durch das medizinische Fachpersonal eingegeben werden.

Beispielsweise kann eine vorgegebene Regel diejenige sein, eine Breite und eine senkrecht dazu stehende Länge der Hautveränderung zu erkennen und eine fusiforme Ellipse zur Verfügung zu stellen, welche mit einem Faktor S, mit vorzugsweise einem Wert des Faktors S zwischen 1,2 bis 2,5, besonders bevorzugt S=1,5, multiplizierte Breite und Höhe aufweist. Vorzugsweise wird bei vorgegebener Breite eine solche Länge der fusiformen Ellipse gewählt, dass der apikale Winkel der fusiformen Ellipse unter 30° beträgt. Insbesondere ist ein Speicher der Bestimmungsvorrichtung dazu angepasst, die vorgegebenen Regeln zu speichern.

Ist eine Naht bzw. Schnittlinie über einer konvexen Körperstelle geplant, wie etwa an den Extremitäten, kann es selbst bei optimaler elliptischer Exzisionsplanung zu Aufwerfungen der Nahtenden kommen. Die Bestimmungsvorrichtung kann in einer Ausführungsform daher unter den vorgegebenen Regeln auch die Regel aufweisen, dass bei konvexen Körperstellen die bestimmten Schnittlinien eine bogen- und/oder S-förmige Schnittlinie (lazy S) aufweisen. Dieses Problem kann das System also durch eine zusätzliche Planung einer bogen- oder S-förmige Konfiguration (lazy S) der Hautnaht vermeiden.

Gemäß einem weiteren Aspekt der Erfindung können die hinterlegten vorgegebenen Regeln eine Regel aufweisen, die bei einer durch die Erfassungsvorrichtung erfassten Hautveränderung mit im Wesentlichen dreieckiger Außenkontur der Hautveränderung eine V-förmige Exzision den Schnittlinien zu Grunde zu legen. Diese weitere Regel kann also bei annähernd dreieckigen Arealen von der Spindelform abweichen mit dem Ziel an der Basis gesundes Gewebe zu sparen und den Schnitt nicht allzu lang erscheinen zu lassen. Dazu bietet sich also die V-förmige Exzision an, die nach lateraler Wundrandmobilisation in Form eines Y zu verschließen ist, was üblicherweise vom Fachmann als VY-Plastik bezeichnet wird.

Gemäß einem weiteren Aspekt der Erfindung können die hinterlegten vorgegebenen Regeln eine Regel aufweisen, die bei einer durch die Erfassungsvorrichtung erfassten Hautveränderung im Gesichtsbereich eine komplexe Verschiebelappenplastik für die zu bestimmenden Schnittlinien, insbesondere eine T-Plastik und/oder U-Plastik und/oder eine Rotationsplastik und/oder ein Verschiebe-Schwenklappen und/oder eine Keilexzision und/oder eine Fernlappenplastik als Kontur der Schnittlinien zugrunde legt.

Insbesondere kann das medizinische Fachpersonal über die Eingabeeinheit eingeben, dass es sich um den Gesichtsbereich handelt, oder die Bestimmungsvorrichtung kann dazu angepasst sein, einen Gesichtsbereich anhand des erfassten Bildes automatisch zu erkennen (Gesichtserkennung). Im Gesichtsbereich können also zur Einhaltung der ästhetischen Einheiten neben der Spindelform auch eine komplexere Verschiebelappenplastik notwendig werden (T-oder U-Plastik, Rotationsplastiken, Verschiebe-Schwenklappen, Keilexzisionen, Fernlappenplastiken oder auch die Entscheidung zur offenen, d.h. sekundären Wundheilung), die abhängig von Anatomie und Größe des Defektes (Hautveränderung) und bis dato abhängig von der Erfahrung des Operateurs, durchgeführt werden kann. Hier kann das System bei der Planung der optimalen Exzisionsform und Simulation verschiedener denkbarer Narbenverläufe bei der Entscheidungsfindung individuell unterstützen.

Gemäß einem weiteren Aspekt der Erfindung können die hinterlegten vorgegebenen Regeln eine Regel aufweisen, die bei einer durch die Erfassungsvorrichtung erfassten Hautveränderung im Hals- und Nacken- Bereich automatisch eine sogenannte doppelte WY-Form als Schnittmuster zu Grunde legt. Des Weiteren sind also gerade im Hals- und Nacken- Bereich auch weitere Schnittformen passend, wie zum Beispiel die doppelte WY-Plastik, wobei der Tumor mit einem doppelten, W-förmigen Schnitt exzidiert wird und ein Y-förmiger Wundverschluss erfolgt. Insgesamt ist das System durch die vorgegebenen und hinterlegten Regeln in der Lage, sämtliche im Stand der Technik und im allgemeinen Fachwissen bekannten Schnittformen entsprechend zu berücksichtigen und bei solchen Schnitten unterstützend zu wirken. Auch können den vorgegebenen, hinterlegten Regeln weitere Regeln hinzugefügt werden, sodass das erfindungsgemäße System entsprechend neuer Forschungsergebnisse ergänzt werden kann.

Vorzugsweise kann die Bestimmungsvorrichtung eine Speichereinheit aufweisen, in welcher eine vordefinierte Datenbank in Form einer Tabelle von vordefinierten Bildern von Hautveränderungen und jeweils zugeordneten Schnittlinien hinterlegt ist, wobei die Bestimmungsvorrichtung das erfasste Bild der Haut des Patienten mit der Hautveränderung mit den vordefinierten Bildern vergleicht und das vordefinierte Bild mit den geringsten Unterschieden zu dem erfassten Bild bestimmt, wodurch, durch die Zuordnung der Schnittlinie zu dem vordefinierten Bild, die Bestimmungsvorrichtung die zugehörigen Schnittlinien bestimmt.

Mit anderen Worten kann in der Speichereinheit eine Datenbank mit zumindest zwei Spalten oder ein zweidimensionales Feld/Array mit je einem grafischen Bild und einem zu dem grafischen Bild zugeordneten Schnittlinie hinterlegt sein. Die Bestimmungsvorrichtung ist dabei insbesondere dazu angepasst, im Einzelvergleich den erfassten Bereich der Haut mit dem grafischen/grafisch hinterlegten Bild zu vergleichen und nach vorgegebenen Regeln eine Abweichung zu bestimmen.

Diese vorgegebenen Regeln können beispielsweise eine mathematische Abweichung von dem Umriss (Form des Umrisses/Kontur) der Hautveränderung des grafischen Bildes zu dem Umriss der erfassten Hautveränderung umfassen. Ebenfalls können die in der Bestimmungsvorrichtung hinterlegen vorgegebenen Regeln derart sein, dass ein Längen-Breiten-Verhältnis und/oder ein apikaler Winkel zwischen dem grafischen Bild und dem erfassten Bereich der Haut des Patienten mit der Hautveränderung verglichen wird und ein Längen-Breiten-Verhältnis und/oder ein apikaler Winkel mit geringstem Unterschied bestimmt wird. Das zumindest zweidimensionale Feld mit dem grafischen Bild, für welches gemäß der vorgegebenen Regeln die geringste Abweichung ermittelt wurde, wird von der Bestimmungsvorrichtung ausgewählt und die dem grafischen Bild zugeordnete Schnittlinie wird als vordefinierte Schnittlinie bestimmt und zur Ausgabe an die Ausgabevorrichtung weitergeleitet.

Gemäß einem weiteren Aspekt der Erfindung kann die Bestimmungsvorrichtung in Abhängigkeit von der Größe der vordefinierten Datenbank eine Gewichtung bei der Berücksichtigung der in der Bestimmungsvorrichtung hinterlegten, vorgegebenen Regeln zur Bestimmung der Schnittlinien oder der vordefinierten Bilder bei der Bestimmung der Schnittlinien vornehmen, insbesondere wobei bei einer kleinen Größe der vordefinierten Datenbank die vorgegebenen Regeln stärker gewichtet werden gegenüber den vordefinierten Bildern in der vordefinierten Datenbank.

Vorzugsweise kann die Bestimmungsvorrichtung dafür angepasst sein, ein computerimplementiertes Verfahren zum Trainieren eines tief gefalteten neuronalen Netzwerks auszuführen, um einen ersten Satz an Bildern von Hautveränderungen mit einem zweiten Satz von Trainingsbildern zu klassifizieren, die durch Vergrößern eines ersten Satzes der Trainingsbilder mittels Drehen des ersten Satzes von Bildern und/oder Drehen des ersten Satzes von Bildern im Farbraum, um Hautfarbvariationen einzuschließen erhalten werden. Vorzugsweise kann die Bestimmungsvorrichtung ferner dafür angepasst sein in dem Verfahren zum Trainieren eine Gewichtung hinsichtlich eines Länge-Breiten-Verhältnisses der Hautveränderung und/oder eines apikalen Winkels in dem tief gefalteten Netzwerk vorzunehmen.

Vorzugsweise kann das System dafür vorgesehen und angepasst sein, die Schritte auszuführen:
- Senden von digitalen Daten einer optischen Aufnahme (Erfassung) der Erfassungsvorrichtung eines Bereichs einer Haut eines Patienten mit einer Hautveränderung an die Bestimmungsvorrichtung;
- vorzugsweise Senden und/oder Übernehmen von Patientendaten an bzw. durch die Bestimmungsvorrichtung oder Laden von Patientendaten, insbesondere durch ein Praxisinformationssystem, ein Bildverarbeitungssystem (PACS) oder die Software eines Dermatoskops, welche in der Speichereinheit der Bestimmungsvorrichtung hinterlegt sind bzw. wird;
- Auswählen einer Exzisionstechnik aus einer Vielzahl von in der Bestimmungsvorrichtung hinterlegten Exzisionstechniken oder durch Anforderung einer manuellen Eingabe durch ein medizinisches Fachpersonal;
- Auswählen einer Schnittform mit chirurgischen Schnittlinien aus einer Vielzahl von hinterlegten Schnittformen (wie etwa einer Spiral-Schnittform, elliptischen Schnittform, kreisrunden Schnittform, W-Schnittform) durch die Bestimmungsvorrichtung;
- Dimensionieren der Schnittform an die Hautveränderung der Hautoberfläche des Patienten durch die Bestimmungsvorrichtung;
- Vorzugsweise Bemaßen der Schnittform durch die Bestimmungsvorrichtung;
- Vorzugsweise Simulation der Rekonstruktion und/oder des Heilungsprozesses durch die Bestimmungsvorrichtung;
- Anforderung einer Eingabe zur Genehmigung der Operationsplanung (der Bestätigung der Auswahl der Schnittform);

Ferner ist das System dafür vorgesehen und angepasst, den nachfolgenden Schritt auszuführen:
- Ausgabe an die Ausgabevorrichtung als Auswahlhilfe für eine passende Schablone aus einem Satz vorgefertigter Schablonen.

Mit anderen Worten wird ein System zur Verfügung gestellt, welches eine Planung und eine Simulation einer Exzision (Schnittlinien) ermöglicht und eine Übertragung des Simulationsergebnisses (Schnittlinien und ggf. weitere operative Informationen) auf die Haut des Patienten gewissermaßen überträgt oder zumindest überlagert ausgibt. Dabei stehen unterschiedliche Möglichkeiten einer Ausgabe zur Verfügung. Das Simulationsergebnis (Schnittlinien) durch die Bestimmungseinheit wird vorzugsweise mittels Auswahl der passenden Schablone aus einem Satz diverser Schablonen und entsprechender Ausgabe durch eine Ausgabevorrichtung das Simulationsergebnis (Schnittlinien) ausgegeben und dargestellt. Zusätzlich kann eine Ausgabe auch mittels einer rechnerunterstützten Zeichenhilfe (insbesondere Augmented Reality) erfolgen. Mit noch anderen Worten wird ein System zur Verfügung gestellt, welches es ermöglicht, rechnergestützt oder computerunterstützt eine dermatologische Exzision anhand einer Bildaufnahme individuell zu planen, zu simulieren und zu optimieren sowie mittels der Vorrichtung oder mittels des Verfahrens diese "Planung" auf eine Haut und das spätere Operationsfeld des Patienten gewissermaßen zu übertragen.

Daneben betrifft die Erfindung ein Verfahren zur Erzeugung und Darstellung auf der Hautoberfläche eines Patienten von vorgegebenen Schnittlinien für die Durchführung eines operativen Eingriffs, mit den Schritten: Senden von digitalen Daten einer optischen Aufnahme eines Bereichs einer Haut eines Patienten mit einer Hautveränderung an eine Bestimmungsvorrichtung; Bestimmen / Zuordnen von Schnittlinien, insbesondere eines Schnittmusters, zu der optischen Aufnahme durch die Bestimmungsvorrichtung; und Ausgeben der bestimmten Schnittlinien, insbesondere eines bestimmten Schnittmusters, durch eine Ausgabevorrichtung, dadurch gekennzeichnet, dass der Schritt des Bestimmens / Zuordnens von Schnittlinien das Bestimmen einer zu verwendenden Schablone aus einem vordefinierten Satz vorgefertigter, eindeutig nummerierter Schablonen umfasst, wobei die zu verwendende Schablone zu den bestimmten vorgegebenen Schnittlinien die geringsten Abweichungen aufweist, und wobei der Schritt des Ausgebens bestimmter Schnittlinien das Anzeigen der eindeutigen Nummer der zu verwendenden Schablone mittels einer Anzeige umfasst. Durch dieses Verfahren kann ein standardisierter und effektiver Prozess zur Erzeugung und Ausgabe von Schnittlinien dem medizinischen Fachpersonal zur Verfügung gestellt werden.

Ebenfalls betrifft die Erfindung ein Computerprogramm, umfassend Befehle, die bei der Ausführung des Programms durch ein erfindungsgemäßes System dieses veranlassen, durch Ausführung der Schritte des erfindungsgemäßen Verfahrens Schnittlinien für eine chirurgische Exzision auf der Hautoberfläche eines Patienten darzustellen.

### Kurzbeschreibung der Figuren

Die Erfindung wird nachfolgend anhand bevorzugter Ausführungsformen mit Hilfe von Figuren näher erläutert. Es zeigen:
- Fig. 1: eine schematische Seitenansicht eines Systems zur Erzeugung und Darstellung von Schnittlinien gemäß eines ersten Vergleichsbeispiels, in welcher das System als portables Modul ausgeführt ist,
- Fig. 2: eine schematische, perspektivische Ansicht eines Systems zur Erzeugung und Darstellung von Schnittlinien gemäß eines zweiten Vergleichsbeispiels, in welcher das System einen Client und einen Server aufweist,
- Fig. 3: eine schematische Ansicht einer Vorlagematrix der Bestimmungseinheit, welche die ausgewählte Schnittform auf eine Hautveränderung anpasst und durch die Ausgabevorrichtung entsprechend anzeigt,
- Fig. 4: eine Teilansicht eines Systems gemäß eines Vergleichsbeispiels, in welcher die Ausgabevorrichtung dazu angepasst ist, mit einem Markierungs-Stift Schnittlinien um eine Hautveränderung herum einzuzeichnen,
- Fig. 5: ein Flussdiagramm eines Arbeitsablaufs eines Systems einer weiteren bevorzugten Ausführungsform,
- Fig. 6: eine schematische Darstellung eines Arbeitsablaufs eines Systems einer weiteren bevorzugten Ausführungsform, in welchem das System einem medizinischen Fachpersonal eine Ausgabeform zur Verfügung stellt und die Schnittlinien durch die Ausgabevorrichtung entsprechend ausgeben lässt,
- Fign. 7 und 8: eine schematische Ansicht einer Benutzeroberfläche eines Systems gemäß einer weiteren Ausführungsform, und

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

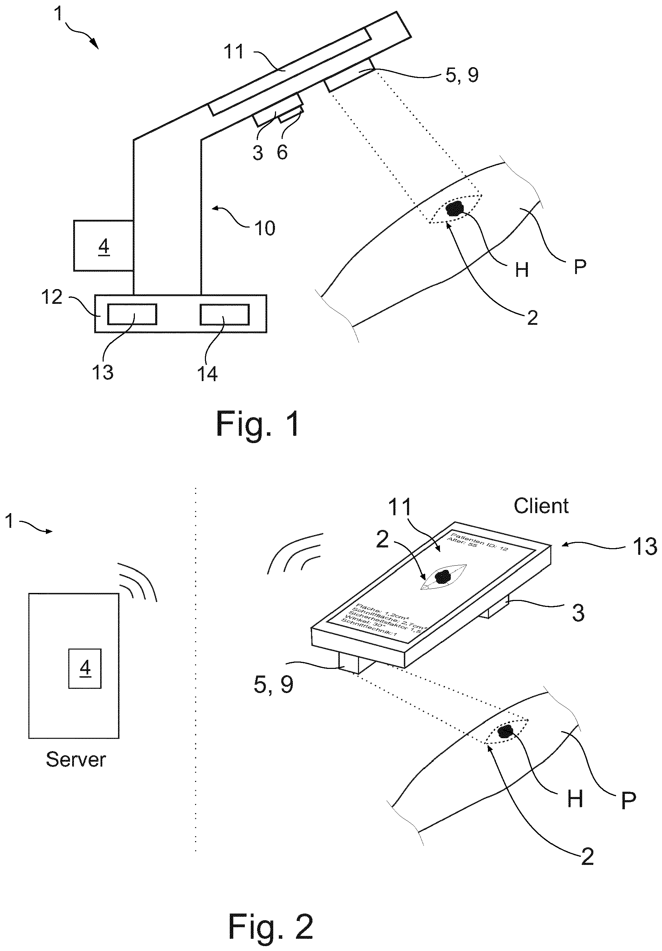

Fig. 1 zeigt in einer schematischen Ansicht ein System 1 zur Erzeugung und Darstellung von Schnittlinien 2 für die Durchführung eines operativen Eingriffs gemäß eines ersten Vergleichbeispiels. Das System 1 ist zur Darstellung dieser vorgegebenen Schnittlinien 2 auf einer Hautoberfläche eines Patienten P, insbesondere von vorgegebenen Schnittlinien 2 für eine chirurgische Exzision angepasst. Der Begriff "Darstellung" ist dabei weit auszulegen und beschränkt sich nicht nur auf eine Darstellung auf etwa einem Display.

Zum digitalen Erfassen, in diesem Vergleichsbeispiel optischen Erfassen, eines Bereichs der Hautoberfläche des Patienten P mit einer Hautveränderung H weist das System eine (elektromagnetische) Erfassungsvorrichtung 3 mit einer (digitalen) Kamera 6 auf, welche im Bereich des sichtbaren Lichts (als elektromagnetische Strahlung) Aufnahmen durchführen kann. Beispielsweise kann die Kamera einen üblichen CMOS Sensor mit vorgeschalteter Optik aufweisen, über welchen ein Bild des Bereichs der Hautoberfläche des Patienten P mit der Hautveränderung H digital erfasst wird.

Das System 1 weist ferner eine Bestimmungsvorrichtung 4 auf. Der von der Erfassungsvorrichtung 3 erfasste Bereich der Haut mit der Hautveränderung H bzw. das erfasste Bild wird anschließend an die Bestimmungsvorrichtung 4 (digital) datentechnisch weitergeleitet. Die Bestimmungsvorrichtung 4 weist in dieser Ausführungsform eine Speichereinheit 7 in Form eines üblichen Speichers, beispielsweise einer Festplatte, einer SSD, eines RAM's o.ä., sowie eine Recheneinheit 8 (ECU) auf, beispielsweise in Form einer CPU und/oder GPU. Die Bestimmungsvorrichtung 4 ist dabei speziell dafür angepasst, anhand des von der Erfassungsvorrichtung 3 erfassten Bilds, (chirurgische) Schnittlinien 2, vorzugsweise ein Schnittmuster/eine Schnittform mit chirurgischen Schnittlinien 2, zur Entfernung der Hautveränderung H zu bestimmen.

Um die Schnittlinien auszugeben und entsprechend darstellen zu können, weist das System 1 ferner eine Ausgabevorrichtung 5 auf, die dafür angepasst ist, die von der Bestimmungsvorrichtung 4 bestimmten Schnittlinien 2 auszugeben und eine Darstellung der Schnittlinien 2 auf der Hautoberfläche zu ermöglichen. In diesem Vergleichsbeispiel weist die Ausgabevorrichtung 5 dafür einen Projektor 9 auf. Beispielsweise kann der Projektor 9 mittels einer Durchlichtprojektion einer digital steuerbaren Anzeigematrix die Schnittlinien 2 auf die Hautoberfläche des Patienten P um die Hautveränderung H herum projizieren. Die Ausgabevorrichtung 5 ist dabei dafür angepasst, mittels des Projektors 9 die Schnittlinien 2 unmittelbar um die Hautveränderung H herum optisch zu projizieren, so dass die Schnittlinien 2 relativ zu der Hautveränderung H exakt so angeordnet werden, wie nachher der tatsächliche operative Schnitt verlaufen soll. Mittels der Erfassungsvorrichtung 3 mit der Kamera 6 wird dabei die Position und Lage des Hautbereichs des Patienten P bzw. der Hautveränderung H räumlich erfasst und entsprechend dieser Position und Lage die Schnittlinien 2 um die Hautveränderung H herum projiziert. Zur Berechnung der angepassten Position und Lage bedient sich die Ausgabevorrichtung in diesem Vergleichsbeispiel der Recheneinheit 8 der Bestimmungsvorrichtung 4. Mit anderen Worten wird durch die Erfassungsvorrichtung 3 der Bereich des Patienten dreidimensional erfasst und der Projektor 9 derart angesteuert, dass relativ zu der Hautveränderung H herum die Schnittlinien angepasst angezeigt (projiziert) werden.

Die Erfassungsvorrichtung 3, die Bestimmungsvorrichtung 4 und die Ausgabevorrichtung 5 sind alle an einer Standbasis 10 angeordnet, so dass dieses System 1 als portables System bzw. Modul ausgestaltet ist und in sich geschlossen verwendet werden kann. Dadurch lässt es sich besonders einfach in Arztpraxen oder Kliniken initial positionieren als auch umpositionieren.

Auf der Rückseite bzw. Oberseite der Standbasis 10 ist ein Touchdisplay 11 angeordnet, über welches ein medizinisches Fachpersonal eine Eingabe, wie etwa Patientendaten durchführen kann als auch Patientendaten oder, alternativ oder zusätzlich zu der Projektion, eine Überlagerung von erfasstem Bild und bestimmten Schnittlinien 2 sich anzeigen lassen kann. Das Touchdisplay 11 übernimmt somit sowohl die Eingabe als auch Ausgabe von Informationen.

An der Unterseite weist die Standbasis 10 einen Standfuß 12 auf, welcher das System 1 auf einer Oberfläche, beispielsweise Tischoberfläche stabil hält. In dem Standfuß 12 sind dabei eine Energieeinheit 13, insbesondere in Form eines Akkumulators wie etwa ein LI-Ionen Akku, sowie eine Kommunikationseinheit 14 mit Antenne (nicht dargestellt) integriert. Die Energieeinheit 13 befähigt das System 1 temporär auch autark ohne Strom mobil einsetzbar zu sein. Mittels der Kommunikationseinheit 14 kann das System auch Daten mit einem Rechner, wie etwa einem Personal Computer oder einem Hand-Held-Device (z.B. Smartphone), austauschen.

Durch das System 1 kann also mithilfe der digitalen optischen Erfassung der Hautveränderung H des jeweiligen Patienten P, eine optimale, personalisierte Schnittform mit Schnittlinien 2 für eine Exzision erstellt und ausgegeben bzw. dargestellt werden. Diese Schnittlinien 2 können dann durch medizinisches Fachpersonal bestätigt werden, und es kann ein operativer Eingriff geplant und simuliert werden. Dem Patienten P kann hierdurch direkt auf seiner betroffenen Hautoberfläche das Schnittmuster mit den Schnittlinien 2 angezeigt werden, was vorteilhaft bei einer präoperativen Aufklärung ist. Des Weiteren ist es mithilfe des Systems 1 möglich, Rekonstruktionen, Nahttechniken, sowie mögliche Wundheilungsverläufe zu simulieren und mit dem Patienten im Vorfeld der möglichen operativen Behandlung zu besprechen.

Fläche, Länge, Winkel und Sicherheitsabstand der zu entfernenden Hautteile und Gewebe werden durch das System 1 bemaßt und auf dem Touchdisplay 11 angezeigt. Ein medizinisches Fachpersonal kann diese Auswahl der Schnittlinien 2 abschließend bewerten.

Im Fall, dass das medizinische Fachpersonal nicht zufrieden mit den durch das System 1 vorgegebenen bzw. bestimmten Schnittlinien 2 ist, kann dieses über das Touchdisplay 11 auf der Benutzeroberfläche eine Eingabe durchführen und insbesondere eine andere Exzisionstechnik und/oder eine unterschiedliche Schnittform wählen, auf Grund dessen die Bestimmungsvorrichtung 4 erneut ein Schnittmuster mit chirurgischen Schnittlinien 2 oder gleich eine Auswahl von mehreren Schnittmustern mit chirurgischen Schnittlinien 2 dem medizinischen Fachpersonal zur Verfügung stellt. Dieses kann das Schnittmuster bestätigen bzw. unter den Schnittmustern das passendste Schnittmuster wählen, welches dann durch für den operativen Eingriff herangezogen wird. Insbesondere kann bei mehreren Schnittmustern mit Schnittlinien 2 bei Auswahl auf dem Touchdisplay 11 dieses direkt auf der Hautoberfläche projiziert werden, so dass das medizinische Fachpersonal und auch der Patient schnell und unkompliziert die direkte Darstellung der Optionen sieht.

Figur 2 zeigt ein System 1 eines weiteren Vergleichsbeispiels. In diesem Vergleichsbeispiel ist das System 1, im Gegensatz zum ersten Vergleichsbeispiel aufgeteilt in einmal einem Handheld 13, der die Erfassungsvorrichtung 3 und die Ausgabevorrichtung 5 aufweist und als Client agiert, sowie separat davon einem Server 14, der die Bestimmungsvorrichtung 4 aufweist. Hierdurch ist es möglich, statt einer 1:1 Beziehung eine 1:n Beziehung von dem zentralen Server 14 mit der Bestimmungseinheit 4, auf welchem insbesondere Daten der Zuordnung von Bild zu Schnittlinien hinterlegt sind, und einer Vielzahl an Clients in Form der Handhelds 13 herzustellen. Insbesondere, wenn eine computerimplementierte Methode zum Training von neuronalen Netzen verwendet wird, kann der zentrale Server 14 zentral die Daten sammeln und zum Training verwenden.

Server 14 und Handheld 13 stehen mittels Netzwerks, in diesem Vergleichsbeispiel über ein drahtloses Netzwerk, insbesondere WLAN Netzwerk, das über das Internet eingebunden sein kann, miteinander in datentechnischer Verbindung und können sowohl Daten empfangen als auch senden.

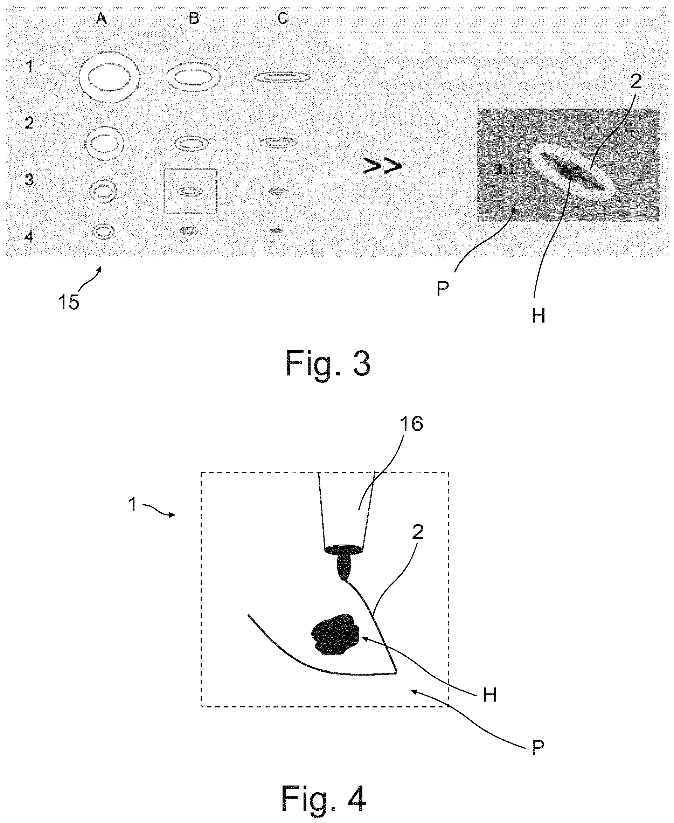

Fig. 3 zeigt schematisch ein in einem System 1 einer bevorzugten Ausführungsform hinterlegte Vorlagematrix 15, die in der Speichereinheit 7 der Bestimmungseinheit 4 gespeichert ist, welche anhand einer Hautveränderung eine auf die Hautveränderung anpasste Schnittform automatisch auswählt oder per Touchdisplay 11 manuell auswählen lässt und durch die Ausgabevorrichtung 5 anzeigen lässt. Beispielsweise wählt das System basierend auf einer Kontur der Hautveränderung des erfassten Bilds automatisch ein Verhältnis Längs zu Breite (A, B oder C; Form) und eine Größe (1, 2 oder 3) aus und gibt diese automatische Auswahl (hier B-3) an die Ausgabevorrichtung weiter. Alternativ oder zusätzlich kann das System auch das medizinische Fachpersonal auffordern, eine Kombination (Buchstabe-Ziffer) auszuwählen, welche dann entsprechend durch die Ausgabevorrichtung ausgegeben wird. So kann ein Nutzer sich auf Hautoberfläche die verschiedenen Schnittlinien bzw. Schnittformen anzeigen lassen und insbesondere manuell schnell ändern.

Fig. 4 zeigt einen Teilausschnitt einer perspektivischen Ansicht eines Systems 1 eines weiteren Vergleichsbeispiels. Das System 1 weist hierbei eine Ausgabevorrichtung 5 mit einer Markierungsvorrichtung 16 mit einem (Markierungs-)Stift auf. Die Markierungsvorrichtung 16 ist dafür angepasst in X-,Y- und Z-Richtung verstellbar ansteuerbar zu sein und durch entsprechende Ansteuerung durch die Ausgabevorrichtung 5 auf der Hautoberfläche des Patienten die Schnittlinien 2 einzuzeichnen. In Fig. 4 ist auch ein apikaler Winkel von kleiner 30° eingezeichnet.

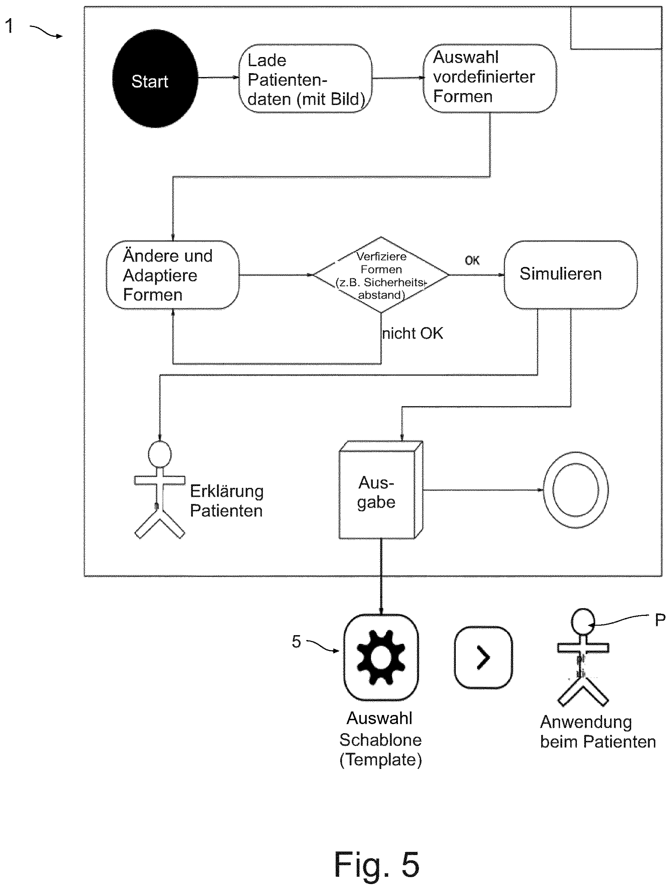

Fig. 5 zeigt in Form eines Flussdiagramms einen Arbeitsablauf des Systems 1 gemäß einer weiteren bevorzugten Ausführungsform. Nach Start des Systems 1 werden zunächst Patientendaten und Aufnahmen der entsprechenden Hautregion mit Hautveränderung H, welche durch die Erfassungsvorrichtung 2 erfasst wurde, geladen. Es stehen verschiedene Schnittformen (beispielsweise Ellipse, W-Y-Plastik und mehr) zur Auswahl, welche durch die Bestimmungsvorrichtung 4 des Systems 1 selbst oder durch ein medizinisches Fachpersonal manuell ausgewählt und in der Größe und Form verändert werden können. Dabei werden chirurgisch relevante Daten (wie etwa Größe, Sicherheitsabstand, usw.) permanent, insbesondere auf einem Display, angezeigt. Nach erfolgreicher Verifikation der Schnittform kann die Exzision und Rekonstruktion, also die Schnittlinien 2, simuliert und dem Patienten erklärt und gezeigt werden. Mit dem Planungsergebnis in Form der Schnittlinien 2 kann dann insbesondere eine passende Schablone aus einem Satz ausgewählt werden und es kann mittels mobilen Gerät (Handheld 13) und mithilfe von Augmented Reality das manuelle Anzeichnen der Schnittplanung der Schnittlinien 2 auf der Haut unterstützt werden.

Sobald also die Planung abgeschlossen und durch den späteren Operateur genehmigt worden ist, können durch das System 1 insbesondere Vorgaben zur Auswahl einer passenden Schablone getroffen werden (aus einem Satz von vorgefertigten Schablonen. Nach Start werden insbesondere Patientendaten und grafische Aufnahmen (erfasste Bilder) der entsprechenden Hautregion mit der Hautveränderung geladen. Es stehen beim System insbesondere verschiedene Schnittformen (z.B.: Ellipse, WY, etc) zur Auswahl, welche ausgewählt und in der Größe und Form verändert werden können. Dabei werden vorzugsweise chirurgisch relevante Daten (z.B.: Größe, Sicherheitsabstand, usw.) permanent angezeigt.

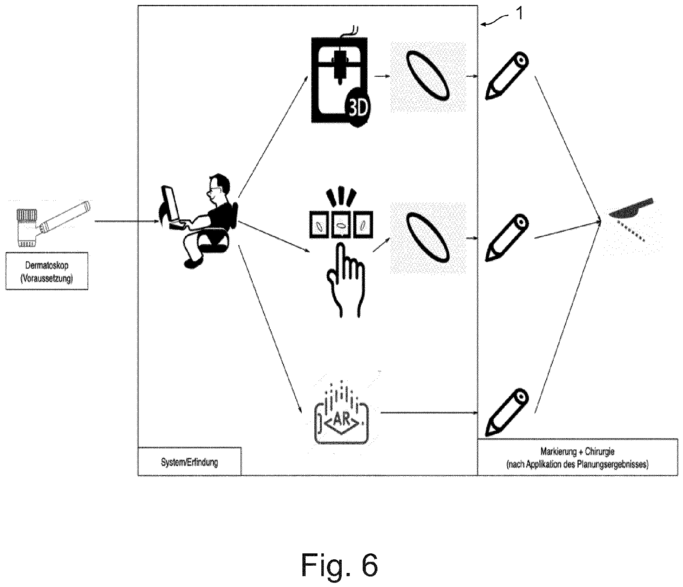

Fig. 6 zeigt in Form eines Flussdiagramms eine Arbeitsweise eines Systems 1 einer weiteren bevorzugten Ausführungsform. In Fig. 6 sind auch die Systemgrenzen des Systems 1 eingezeichnet. Eine Aufnahme der betroffenen Hautregion kann beispielsweise im Vorfeld mittels digitalem Dermatoskop erfolgen. Entscheidend ist für das System 1 nur, dass ein erfasstes Bild, insbesondere digitales Bild, von der Hautoberfläche des Patienten mit der Hautveränderung vorliegt. In dieser Ausführungsform ist die Erfassungsvorrichtung dafür angepasst, ein bereitgestelltes (digitales) Bild, was beispielsweise schon vor einigen Tagen durch einen Arzt aufgenommen wurde, zu erfassen, zu verarbeiten und durch die Ausgabevorrichtung 5 entsprechende bestimmte Schnittlinien 2 zur Verfügung zu stellen, hier beispielsweise in Form von einer ausgedruckten Schablone. Die eigentliche Chirurgie kann dann unabhängig von dem System 1 und der Ausgabevorrichtung 5, insbesondere nur mittels der bereitgestellten, individuellen Schablone, durchgeführt werden.

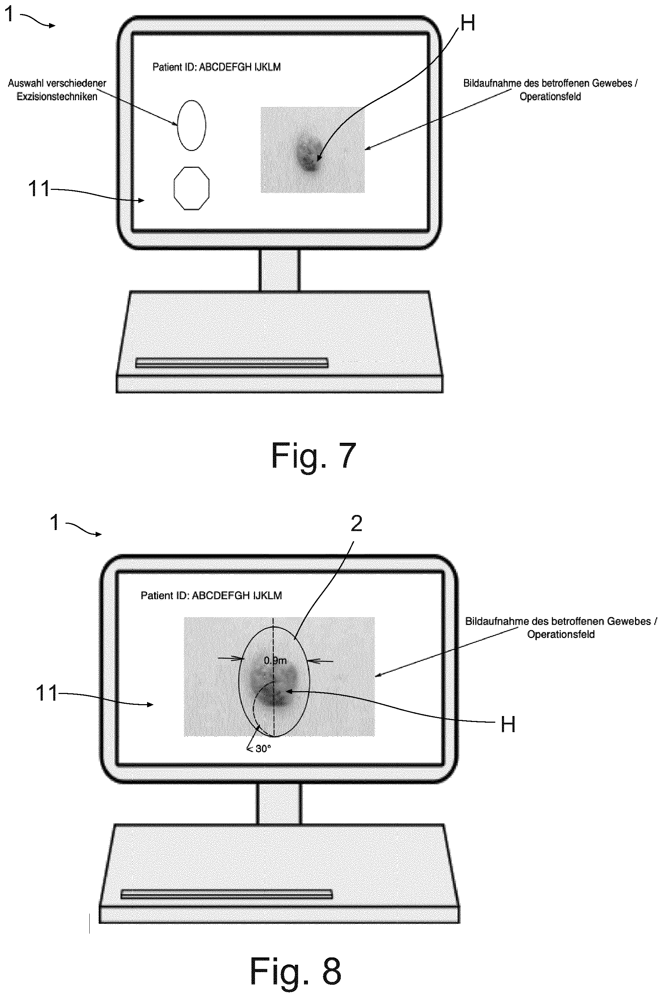

Fign. 7 und 8 zeigt schematisch eine Benutzeroberfläche eines Systems 1 einer weiteren bevorzugten Ausführungsform, welche über ein Display oder Touchdisplay angezeigt wird. Auf der Benutzeroberfläche wird nach dem Ladevorgang eines bestimmten Patienten und dem Laden des erfassten Bilds bzw. der Aufnahme des betroffenen Hautbereichs, verschiedene Exzisionstechniken und Schnittformen angezeigt, welche ausgewählt und modifiziert werden können.

Fig. 8 stellt schematisch dar, wie nach Auswahl einer bestimmten Exzisiontechnik und Schnittform (wie etwa Ellipse), das betroffene Gewebe (beispielsweise ein Nävus) umrandet und die Schnittfläche bemaßt wird. Dabei werden insbesondere Maße bzw. Parameter wie Gesamtfläche und/oder Länge und/oder Schnittwinkel permanent angezeigt, welche von dem medizinischen Fachpersonal abgelesen werden können.

### Bezugszeichenliste

1 System zur Erzeugung und Darstellung von Schnittlinien
2 Schnittlinie
3 Erfassungsvorrichtung
4 Bestimmungsvorrichtung
5 Ausgabevorrichtung
6 Kamera
7 Speichereinheit
8 Recheneinheit
9 Projektor
10 Standbasis
11 Touchdisplay
12 Standfuß
13 Handheld
14 Server
15 Vorlagematrix
16 Markierungsvorrichtung
P Patient
H Hautveränderung

## Patentansprüche

1. System (1) zur Erzeugung von Schnittlinien (2) für die Durchführung eines operativen Eingriffs und Darstellung dieser vorgegebenen Schnittlinien (2) auf der Hautoberfläche eines Patienten (P), insbesondere von vorgegebenen Schnittlinien (2) für eine chirurgische Exzision, umfassend:
eine elektromagnetische Erfassungsvorrichtung (3) zum digitalen Erfassen, vorzugsweise optischen Erfassen, eines Bereichs der Hautoberfläche eines Patienten (P) mit einer Hautveränderung (H),
eine Bestimmungsvorrichtung (4), die dafür angepasst ist, anhand des von der Erfassungsvorrichtung (3) erfassten Bereichs der Haut mit der Hautveränderung (H) Schnittlinien (2), vorzugsweise ein Schnittmuster/eine Schnittform mit chirurgischen Schnittlinien (2), zur Entfernung der Hautveränderung (H) zu bestimmen, und
eine Ausgabevorrichtung (5), die dafür angepasst ist, die von der Bestimmungsvorrichtung (4) bestimmten Schnittlinien (2) auszugeben und eine Darstellung der Schnittlinien (2) auf der Hautoberfläche zu ermöglichen,
**dadurch gekennzeichnet, dass** das System (1) ferner einen vordefinierten Satz an vorgefertigten, eindeutig nummerierten Schablonen aufweist, wobei:
die Bestimmungsvorrichtung (4) ferner dafür angepasst ist, aus dem Satz der nummerierten Schablonen die zu verwendende Schablone zu bestimmen, welche zu den bestimmten vorgegebenen Schnittlinien (2) die geringsten Abweichungen aufweist, und
die Ausgabevorrichtung (5) dafür angepasst ist, mittels einer Anzeige die eindeutige Nummer der zu verwendenden Schablone anzuzeigen.

2. System (1) nach Anspruch 1, **dadurch gekennzeichnet, dass** die vorgefertigten, eindeutig nummerierten Schablonen auf den erfassten Bereich der Haut mit der Hautveränderung auflegbar und insbesondere auf dem Bereich der Haut fixierbar sind.

3. System (1) nach einem der vorangegangenen Ansprüche, **dadurch gekennzeichnet, dass** das System ferner eine Eingabevorrichtung (11), insbesondere in Form einer Tastatur, aufweist, die dafür angepasst ist, weitere medizinische Parameter des Patienten zu erfassen, diese Parameter an die Bestimmungsvorrichtung (4) weiterzuleiten, wobei die Bestimmungsvorrichtung (4) diese Parameter bei der Bestimmung der Schnittlinien (2) berücksichtigt.

4. System (1) nach einem der vorangegangenen Ansprüche, **dadurch gekennzeichnet, dass** ein portabler Client (13) die Erfassungsvorrichtung (3) und/oder die Ausgabevorrichtung (5) aufweist und ein zentraler Server (14) die Bestimmungsvorrichtung (4) aufweist, wobei die Erfassungsvorrichtung (3), die Ausgabevorrichtung (5) und die Bestimmungsvorrichtung (4) über ein Netzwerk, insbesondere LAN- oder WLAN-Netzwerk oder über das Internet, miteinander in Verbindung stehen, um digitale Daten auszutauschen.

5. System (1) nach einem der vorangegangenen Ansprüche, **dadurch gekennzeichnet, dass** die Bestimmungsvorrichtung (4) zur Bestimmung der Schnittlinien (2) vorgegebene Regeln anwendet, um anhand des von der Erfassungsvorrichtung (3) erfassten Bereichs der Haut mit der Hautveränderung Schnittlinien (2) zur Entfernung der Hautveränderung zu bestimmen.

6. System (1) nach einem der vorangegangenen Ansprüche, **dadurch gekennzeichnet, dass** die Bestimmungsvorrichtung (4) eine Speichereinheit aufweist, in welcher eine vordefinierte Datenbank in Form einer Tabelle von vordefinierten Bildern von Hautveränderungen und jeweils zugeordneten Schnittlinien (2) hinterlegt ist, wobei die Bestimmungsvorrichtung (4) das erfasste Bild der Haut des Patienten mit der Hautveränderung mit den vordefinierten Bildern vergleicht und das vordefinierte Bild mit den geringsten Unterschieden bestimmt, wodurch die Bestimmungsvorrichtung (4) die zugehörigen Schnittlinien (2) bestimmt.

7. System (1) nach Anspruch 6, **dadurch gekennzeichnet, dass** die Bestimmungsvorrichtung (4) in Abhängigkeit von der Größe der vordefinierten Datenbank eine Gewichtung bei der Berücksichtigung der in der Bestimmungsvorrichtung (4) hinterlegten, vorgegebenen Regeln zur Bestimmung der Schnittlinien (2) oder der vordefinierten Bilder bei der Bestimmung der Schnittlinien (2) vornimmt, insbesondere wobei bei einer kleinen Größe der vordefinierten Datenbank die vorgegebenen Regeln stärker gewichtet werden gegenüber den vordefinierten Bildern in der vordefinierten Datenbank.

8. Verfahren zur Erzeugung und Darstellung auf der Hautoberfläche eines Patienten von vorgegebenen Schnittlinien (2) für die Durchführung eines operativen Eingriffs, mit den Schritten:
Senden von digitalen Daten einer optischen Aufnahme eines Bereichs einer Haut eines Patienten mit einer Hautveränderung an eine Bestimmungsvorrichtung (4),
Bestimmen / Zuordnen von Schnittlinien (2), insbesondere eines Schnittmusters, zu der optischen Aufnahme durch die Bestimmungsvorrichtung (4), und
Ausgeben bestimmter Schnittlinien (2), insbesondere eines bestimmten Schnittmusters, durch eine Ausgabevorrichtung (5),
**dadurch gekennzeichnet, dass** der Schritt des Bestimmens / Zuordnens von Schnittlinien (2) das Bestimmen einer zu verwendenden Schablone aus einem vordefinierten Satz vorgefertigter, eindeutig nummerierter Schablonen umfasst, wobei die zu verwendende Schablone zu den bestimmten vorgegebenen Schnittlinien (2) die geringsten Abweichungen aufweist, und
wobei der Schritt des Ausgebens bestimmter Schnittlinien (2) das Anzeigen der eindeutigen Nummer der zu verwendenden Schablone mittels einer Anzeige umfasst.

9. Computerprogramm, umfassend Befehle, die bei der Ausführung des Programms durch ein System wie in einem der Ansprüche 1 bis 7 definiert dieses veranlassen, durch Ausführung der Schritte wie in Anspruch 8 definiert Schnittlinien (2) für eine chirurgische Exzision auf der Hautoberfläche eines Patienten (P) darzustellen.

## Claims

1. System (1) for generating cutting lines (2) for performing a surgical intervention and displaying these predetermined cutting lines (2) on the skin surface of a patient (P), in particular predetermined cutting lines (2) for a surgical excision, comprising:
an electromagnetic detection device (3) for digitally detecting, preferably optically detecting, an area of the skin surface of a patient (P) with a skin alteration (H),
a determination device (4) adapted to determine cutting lines (2), preferably a cutting pattern/cutting shape with surgical cutting lines (2), for removing the skin alteration (H) on the basis of the area of the skin with the skin alteration (H) detected by the detection device (3), and
an output device (5) adapted to output the cutting lines (2) determined by the determination device (4) and to enable visualisation of the cutting lines (2) on the skin surface, **characterised in that** the system (1) further comprises a predefined set of prefabricated, distinctly numbered templates, wherein:
the determining device (4) is further adapted to identify from the set of numbered templates the template to be used having the least deviations from the determined predetermined cutting lines (2), and
the output device is adapted to indicate, by means of a display, the unique number of the template to be used.

2. System (1) according to claim 1, **characterised in that** the prefabricated, distinctly numbered templates can be placed on the detected area of the skin with the skin alteration and, in particular, can be fixed to the area of the skin.

3. System (1) according to one of the preceding claims, **characterised in that** the system further comprises an input device (11), in particular in the form of a keyboard, which is adapted to record further medical parameters of the patient, to forward these parameters to the determination device (4), the determination device (4) taking these parameters into account when determining the cutting lines (2).

4. System (1) according to one of the preceding claims, **characterised in that** a portable client (13) comprises the detection device (3) and/or the output device (5), and a central server (14) comprises the determination device (4), wherein the detection device (3), the output device (5) and the determination device (4) are connected to one another via a network, in particular a LAN or WLAN network or via the internet, in order to exchange digital data.

5. System (1) according to one of the preceding claims, **characterised in that** the determination device (4) for determining the cutting lines (2) applies predetermined rules in order to determine cutting lines (2) for removing the skin alteration on the basis of the area of the skin with the skin alteration detected by the detection device (3).

6. System (1) according to one of the preceding claims, **characterised in that** the determination device (4) has a memory unit in which a predefined database is stored in the form of a table of predefined images of skin alterations and associated cutting lines (2), wherein the determination device (4) compares the captured image of the patient's skin showing the skin alteration with the predefined images and determines the predefined image with the smallest differences, whereby the determination device (4) determines the associated cutting lines (2).

7. System (1) according to claim 6, **characterised in that** the determination device (4), depending on the size of the predefined database, carries out a weighting when taking into account the predefined rules stored in the determination device (4) for determining the cutting lines (2) or the predefined images when determining the cutting lines (2), in particular the predefined rules being weighted more heavily than the predefined images in the predefined database if the predefined database is small.

8. Method for generating and displaying on a patient's skin surface predetermined cutting lines (2) for performing a surgical intervention, comprising the steps of:
sending digital data of an optical image of an area of a patient's skin with a skin alteration to a determination device (4),
determining/assigning cutting lines (2), in particular a cutting pattern, to the optical image by the determination device (4), and
delivering defined cutting lines (2), in particular a defined cutting pattern, by an output device (5),
**characterised in that** the step of determining/assigning cutting lines (2) comprises determining a template to be used from a predefined set of prefabricated, distinctly numbered templates, wherein the template to be used has the smallest deviations from the assigned predetermined cutting lines (2), and
wherein the step of delivering defined cutting lines (2) comprises displaying the unique number of the template to be used by means of a display.

9. Computer program comprising instructions which, when the program is executed by a system as defined in any one of claims 1 to 7, cause it to display cutting lines (2) for a surgical excision on the skin surface of a patient (P) by executing the steps as defined in claim 8.

## Revendications

1. Système (1) pour la génération de lignes de coupe (2) pour la réalisation d'une intervention chirurgicale et la représentation de ces lignes de coupe (2) prédéfinies sur la surface de la peau d'un patient (P), notamment de lignes de coupe (2) prédéfinies pour une excision chirurgicale, comprenant :
un dispositif d'acquisition électromagnétique (3) pour l'acquisition numérique, de préférence l'acquisition optique, d'une zone de la surface de la peau d'un patient (P) présentant une altération de la peau (H),
un dispositif de détermination (4) adapté pour déterminer, à partir de la zone de la peau présentant l'altération de la peau (H) acquise par le dispositif d'acquisition (3), des lignes de coupe (2), de préférence un motif de coupe/une forme de coupe avec des lignes de coupe chirurgicales (2), pour enlever l'altération de la peau (H), et
un dispositif de sortie (5) adapté pour sortir les lignes de coupe (2) déterminées par le dispositif de détermination (4) et pour permettre une représentation des lignes de coupe (2) sur la surface de la peau,
**caractérisé en ce que** le système (1) présente en outre un ensemble prédéfini de gabarits préfabriqués, numérotés de manière unique :
le dispositif de détermination (4) étant en outre adapté pour déterminer, à partir de l'ensemble des gabarits numérotés, le gabarit à utiliser qui présente les écarts les plus faibles par rapport aux lignes de coupe prédéfinies déterminées (2), et
le dispositif de sortie (5) étant adapté pour indiquer, au moyen d'un affichage, le numéro unique du gabarit à utiliser.

2. Système (1) selon la revendication 1, **caractérisé en ce que** les gabarits préfabriqués, numérotés de manière unique, peuvent être appliqués sur la zone acquise de la peau présentant l'altération de la peau et peuvent notamment être fixés sur la zone de la peau.

3. Système (1) selon l'une quelconque des revendications précédentes, **caractérisé en ce que** le système présente en outre un dispositif de saisie (11), notamment sous la forme d'un clavier, adapté pour acquérir d'autres paramètres médicaux du patient, transmettre ces paramètres au dispositif de détermination (4), le dispositif de détermination (4) prenant ces paramètres en compte lors de la détermination des lignes de coupe (2).

4. Système (1) selon l'une quelconque des revendications précédentes, **caractérisé en ce qu'**un client portable (13) présente le dispositif d'acquisition (3) et/ou le dispositif de sortie (5) et un serveur central (14) présente le dispositif de détermination (4), le dispositif d'acquisition (3), le dispositif de sortie (5) et le dispositif de détermination (4) étant en communication les uns avec les autres via un réseau, notamment un réseau LAN ou WLAN ou via Internet, pour échanger des données numériques.

5. Système (1) selon l'une quelconque des revendications précédentes, **caractérisé en ce que** le dispositif de détermination (4) pour déterminer les lignes de coupe (2) utilise des règles prédéfinies pour déterminer, à partir de la zone de la peau présentant l'altération de la peau acquise par le dispositif d'acquisition (3), des lignes de coupe (2) pour enlever l'altération de la peau.

6. Système (1) selon l'une quelconque des revendications précédentes, **caractérisé en ce que** le dispositif de détermination (4) présente une unité de mémoire dans laquelle est stockée une base de données prédéfinie sous la forme d'un tableau d'images prédéfinies d'altérations de la peau et de lignes de coupe (2) respectivement associées, le dispositif de détermination (4) comparant l'image acquise de la peau du patient présentant l'altération de la peau avec les images prédéfinies et déterminant l'image prédéfinie présentant les différences les plus faibles, ce qui permet au dispositif de détermination (4) de déterminer les lignes de coupe (2) correspondantes.

7. Système (1) selon la revendication 6, **caractérisé en ce que** le dispositif de détermination (4) effectue, en fonction de la taille de la base de données prédéfinie, une pondération lors de la prise en compte des règles prédéfinies stockées dans le dispositif de détermination (4) pour la détermination des lignes de coupe (2) ou des images prédéfinies lors de la détermination des lignes de coupe (2) ; notamment, lorsque la taille de la base de données prédéfinie est petite, les règles prédéfinies étant pondérées plus fortement que les images prédéfinies dans la base de données prédéfinie.

8. Procédé de génération et de représentation sur la surface de la peau d'un patient de lignes de coupe (2) prédéfinies pour la réalisation d'une intervention chirurgicale, avec les étapes suivantes :
l'envoi de données numériques d'un enregistrement optique d'une zone d'une peau d'un patient présentant une altération de la peau à un dispositif de détermination (4),
la détermination/l'association de lignes de coupe (2), notamment d'un motif de coupe, à l'enregistrement optique par le dispositif de détermination (4), et
la sortie de lignes de coupe déterminées (2), notamment d'un motif de coupe déterminé, par un dispositif de sortie (5),
**caractérisé en ce que** l'étape de détermination/association de lignes de coupe (2) comprend la détermination d'un gabarit à utiliser à partir d'un ensemble prédéfini de gabarits préfabriqués numérotés de manière unique, le gabarit à utiliser présentant les écarts les plus faibles par rapport aux lignes de coupe prédéfinies déterminées (2), et
l'étape de sortie de lignes de coupe déterminées (2) comprenant l'affichage du numéro unique du gabarit à utiliser au moyen d'un affichage.

9. Programme informatique comprenant des instructions qui, lors de l'exécution du programme par un système tel que défini dans l'une quelconque des revendications 1 à 7, amènent celui-ci à représenter des lignes de coupe (2) pour une excision chirurgicale sur la surface de la peau d'un patient (P) en exécutant les étapes telles que définies dans la revendication 8.
